# EUROPEAN PATENT APPLICATION

(11) **EP 1 403 795 A1**
(43) Date of publication of application: **31.03.2004**
(21) Application number: 02741204.8
(22) Date of filing: 19.06.2002
(51) Int. Cl.: G06F 17/60, H04M 11/00, G06F 9/06, G08C 19/00, G01N 33/48

(54) **INFORMATION COMMUNICATION SYSTEM**

(30) Priority: 22.06.2001 JP 2001189104
(71) Applicant: ARKRAY, Inc., Kyoto-shi, Kyoto 601-8045 (JP)
(72) Inventor: KAWATAHARA, Masanao c/o ARKRAY, Inc., Kyoto-shi, Kyoto 601-8045 (JP); OOURA, Yoshimi c/o ARKRAY, Inc., Kyoto-shi, Kyoto 601-8045 (JP); DOI, Shigeru c/o ARKRAY, Inc., Kyoto-shi, Kyoto 601-8045 (JP)
(74) Representative: Pluckrose, Anthony William
(86) International application number: PCT/JP2002/006135
(87) International publication number: WO 2003/001423

(57) **Abstract**

The present invention relates to an information communication system (X) including a storage medium (4) in which target information is stored in advance, an information processor (1) for inputting the target information from the storage medium (4), and an analyzer (2) for inputting the target information from the information processor (4). In the information communication system (X) , the target information from the storage medium (4) is inputted into the analyzer (2) through the information processor (1). Preferably, the information communication system (X) further includes an additional information processor (3) into which information from the analyzer (2) is inputted and which performs a predetermined operation in accordance with the information. The target information includes a program for causing the information from the analyzer (2) to correspond to the communication protocol of the additional information processor (3) before the information is outputted.

## Description

### TECHNICAL FIELD

The present invention relates to an information communication system in which information from a storage medium is inputted into an analyzer via an information processing apparatus.

### BACKGROUND ART

It is important for diabetics to regularly check their own blood glucose level for controlling the blood glucose level. However, it is troublesome to frequently visit a medical institution for measuring the blood glucose level. In light of this, portable handheld blood glucose level measuring apparatuses are used, whereby diabetics can easily and conveniently measure the blood glucose level even when they are away from home, for example.

Basically, such a portable blood glucose level measuring apparatus is used for controlling the blood glucose level by diabetics themselves based on the measurement results. However, even in such a case, it is preferable to regularly receive professional instructions from a doctor or a specialist, for example. For receiving instructions, the measurement results may need to be shown regularly to a specialist. For this purpose, a hard copy of the measurement data may need to be handed to the specialist, or the blood glucose level measuring apparatus may need to be brought to the medical institution to enable the specialist to access the data. After all, such methods also necessitate a visit to the medical institution, which is troublesome.

To avoid the above trouble, the measurement data stored in the blood glucose level measuring apparatus may be transmitted to a computer of the medical institution through telecommunication lines. However, a typical portable blood glucose level measuring apparatus does not have the function for telecommunications. Therefore, the blood glucose level measuring apparatus may be connected to a mobile communicator (e.g. a cell phone) for inputting data into the mobile communicator and transmitting the data to a computer of a medical institution by e-mail, for example.

Generally, however, the communication protocol of the portable blood glucose level measuring apparatus is different from that of the mobile communicator, and neither the portable blood glucose level measuring apparatus nor the mobile communicator can change its communication protocol. Therefore, for transmitting measurement data or the like via the mobile communicator, the measurement data needbe converted into data with a format corresponding to the communication protocol of the mobile communicator. For this purpose, the portable blood glucose level measuring apparatus and the mobile communicator need be connected to each other via a personal computer in which a program for changing the data format is installed, so that the data format change is performed by the personal computer. Therefore, every time the measurement data is to be transmitted, the transmitter needs to connect the portable blood glucose level measuring apparatus to the personal computer and to the mobile communicator. When the personal computer to be used is a desktop computer, transmission of measurement data is impossible when the transmitter is away from home, for example. In this way, with the portable blood glucose level measuring apparatus, transmitting and receiving of data to and from an external device cannot be performed readily unless the program related to the communication protocol is changed.

### DISCLOSURE OF THE INVENTION

An object of the present invention is to facilitate the data transmission between an analyzer and an information processor.

According to a first aspect of the present invention, there is provided an information communication system comprising a storage medium in which target information is stored in advance, an information processor for inputting the target information from the storage medium, and an analyzer for inputting the target information from the information processor. The target information from the storage medium is inputted into the analyzer through the information processor.

The information processor may be a personal computer, a set-top box or a mobile communicator (e.g. a cell phone), for example.

The storage medium may be a database accessible through telecommunication lines, an optical disk or a magnetic disk, for example. As an example of method of reading the target information from a database accessible through telecommunication lines and inputting the information into the information processor, the user may access a homepage on the Internet and download the target information from the homepage. The telecommunication may be wired or wireless. The optical disk herein means a disk which can reproduce information by utilizing light, and examples of such disk include a CD, a DVD, a magneto optical disk and a phase change disk.

Preferably, the information communication system according to the present invention further includes an additional information processing system into which information from the analyzer is inputted and which performs a predetermined operation in accordance with the information.

In this case, the target information may be information necessary for realizing the operation of the additional information processor. The target information may include a program for causing the information outputted from the analyzer to correspond to the communication protocol of the additional information processor before the information is outputted.

Examples of additional information processor include a mobile communicator (e.g. a cell phone) , a PDA, a personal computer, a portable game machine, a home-use game machine and a printer. The PC or the printer may belong to an individual who is the owner (user) of the analyzer or may belong to a medical institution managing the blood glucose level of a patient.

The information communication system according to the present invention may include a plurality of additional information processors or a single information processor. The information processor and the additional information processor may be realized by the same apparatus (when a single information processor functions as both of the information processor and the additional information processor).

In the case where the information communication system includes an additional information processor, the target information may be (1) information which is selected in accordance with information newly stored in the analyzer within a certain time period and which contributes to the game property of the game machine when the additional information processor is a game machine, (2) a calculation program necessary for the analysis of an object at the analyzer and (3) a program for realizing a function which is necessary for the operation of the analyzer but is not directly related with the calculation required for the analysis of an object.

The analyzer of the present invention may be a desktop device or a portable device.

The analyzer may include a rewritable memory. In that case, the target information may be stored in the memory by overwriting the content of the memory. Alternatively, the target information may be additionally stored in the memory.

The analyzer may measure the concentration of a particular component in a measurement object. A typical example of such analyzer is an apparatus for measuring the glucose concentration in blood. Alternatively, as the analyzer, use may be made of an apparatus for measuring cholesterol in blood as well as an apparatus for measuring biological information in a noninvasive manner. Examples of apparatus for measuring biological information in a noninvasive manner include a noninvasive blood glucose level measuring apparatus, a pulseoximeter, an electrocardiograph, a sphygmomanometer, a pulse meter as well as an apparatus for examining water and an apparatus for measuring a component in food.

The analyzer may include a main body, and a detection unit removably attached to the main body for outputting information to the main body. In this case, the main body includes a calculating section for performing calculation necessary for analyzing a particular component contained in a measurement object, and the detection unit includes a detection section for obtaining calculation information necessary for the calculation from the measurement object.

In the analyzer, a new detection unit can be used instead of the previously used one. Alternatively, a new detection unit or units can be used in addition to the previously used one. In that case, the detection unit suitable for the intended purpose may be selected for use from a plurality of detection units.

The detection section may function to provide stimulation for the measurement object and obtain the calculation information as a response to the stimulation.

Examples of stimulation include light, voltage and electric current, whereas examples of response include light, electric current and voltage. When the response is light, potential in accordance with the amount of response can be obtained as transmittance, reflectivity or absorbency. The detection section may obtain calculation information by an electrochemical method, an optical method or a noninvasive method.

The detection unit may have a structure which allows mounting of an analyzing tool for retaining the measurement object. The analyzing tool may be one that provides a reaction system for causing enzyme reaction, chemical reaction or immune reaction to occur. The analyzing tool may be a biosensor or a test piece having a color-developing portion, for example. Alternatively, the detection unit may utilize an analyzing tool that outputs calculation information by a non-reactive method (e.g. an ion-selective plate).

The main body may further include a memory for storing a program necessary for the calculation. In this case, the memory may store a plurality of programs corresponding to a plurality of measurement items or a single program corresponding to a single item.

When a new detection unit or units are to be used additionally or instead of the previously used one, a calculation program appropriate for the new detection unit may not be prestored in the memory. In that case, the main body further includes an inputting section for inputting a new calculation program from an external device. The new calculation program inputted via the inputting section may be stored in the memory, for example. For storing a new calculation program in the memory, the content of the existing calculation program may be changed or another calculation program may be additionally stored in the memory. The manner of storing may be selected depending on the capacity which the memory originally had or the remaining capacity of the memory at the time of storing.

Preferably, the main body may further include a detection-unit-identifying section for identifying the type of the mounted detection unit. The detection-unit-identifying section may identify the type of the detection unit in accordance with the operation of a button by the user or may automatically identify the type of the detection unit when the detection unit is mounted to the main body. In the latter case, information corresponding to the type of the detection unit is transmitted from the detection unit to the identifying section, whereby the identifying section can identify the type of the detection unit.

Preferably, the main body may further include an install requesting section for requesting installation of a new calculation program based on the identification by the detection-unit-identifying section. Such a request may be indicated by a lamp such as an LED or displayed on the display provided for showing analysis results, whereby the user can notice the necessity for installing a new calculation program.

### BRIEF DESCRIPTION OF THE DRAWINGS

Fig. 1 is a system chart illustrating an example of information communication system according to the present invention.
Fig. 2 is a perspective view illustrating an example of analyzer.
Fig. 3 is a block diagram of the analyzer shown in Fig. 2.
Fig. 4 is a plan view showing an example of a display showing a request for installation of a program.
Fig. 5 is a perspective view illustrating an example of manner of using the analyzer.
Figs. 6A and 6B each is a system chart illustrating a manner of installing a program in the analyzer.

### BEST MODE FOR CARRYING OUT THE INVENTION

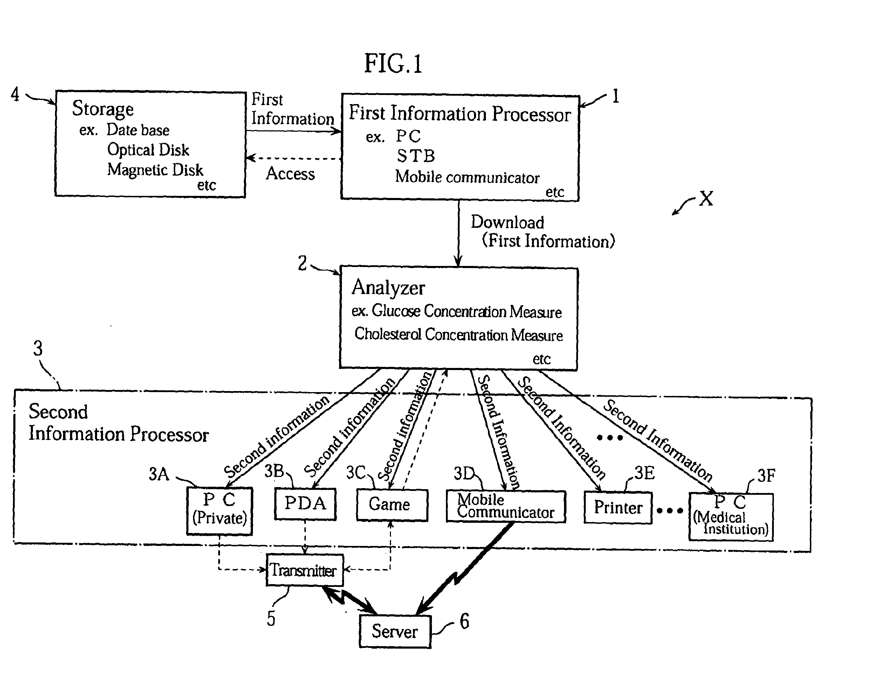

As shown in Fig. 1, an information communication system X according to the present invention may include a first information processor 1, an analyzer 2 and several kinds of second information processors 3.

In the information communication system X, target information can be downloaded from a storage medium 4 into the analyzer 2 through the first information processor 1. By the downloading, a new function may be added to the analyzer 2, or the original function of the analyzer 2 may be changed. Further, a second information processor 3A-3F, which is selected from the second information processors 3 of the different types, may be made usable for the intended purpose in accordance with second information from the analyzer 2.

The first information processor 1 includes a rewritable memory (not shown) comprising a RAM, for example. This memory stores first information read from the storage medium 4. The first information processor 1 causes the first information stored in the memory to be downloaded into the analyzer 2. As the first information processor 1 having the above function, use may be made of a personal computer (PC), a set-top box (STB) or a mobile communicator (e.g. a cell phone), for example. The first information processor 1 may be owned by the user of the analyzer 2, or by a manufacturer/distributor or consignor of the analyzer 2. Thus, the first information may be downloaded into the analyzer 2 by the owner himself or herself of the analyzer 2. Alternatively, the first information may be downloaded into the analyzer 2 by the manufacturer/distributor or consignor (serviceman) of the analyzer 2.

As the storage medium 4, use may be made of a database accessible through telecommunication lines, an optical disk or a magnetic disk, for example. The telecommunication may be wired or wireless. A "database" herein may refer not only to the one provided chiefly for data-collecting purposes, but also to a homepage established on the Internet. The owner or establisher of the database may be the manufacturer/distributor or consignor of the analyzer 2 or they may be a medical institution, for example. An "optical disk" herein refers to a disk which can reproduce information by light, including a CD, a DVD, a magneto optical disk and a phase change disk.

The analyzer 2 may measure the concentration of a particular component (e.g. glucose or cholesterol) in a measurement obj ect such as a biochemical sample (e.g. blood, urine or saliva). Typically, the analyzer 2 is a portable device. As the analyzer 2, use may be made of an apparatus designed to measure biological information in a noninvasive manner. Examples of noninvasive apparatus for measuring biological information include a noninvasive blood glucose level measuring apparatus, a pulseoximeter, an electrocardiograph, a sphygmomanometer, a pulse meter as well as an apparatus for examining water and an apparatus for measuring a component in food.

The analyzer 2 includes storage (not shown) for storing the first information. Part or the entirety of the storage may be provided by a volatile memory. That is, the storage may be provided solely by a volatile memory such as a RAM, or by both a volatile memory and a nonvolatile memory such as a ROM.

As noted above, the first information stored in the first information processor 1 is downloaded into the memory of the analyzer 2. At this time, the first information may be downloaded after part of the stored data is deleted, or it may be downloaded into an empty space if the capacity of the memory is sufficient.

The second information processor 3 is used for a predetermined purpose in accordance with the second information from the analyzer 2. Examples of second information processor 3 include a personal computer (PC) 3A owned by a particular person, a portable terminal (PDA) 3B, a game machine 3C, a mobile communicator 3D, a printer 3E and a PC 3F owned by a medical institution. The memory of the analyzer 2 has a program installed for enabling at least one second information processor selected from the apparatuses 3A-3F to process the second information from the analyzer 2. By this program, the selected one of the second information processors 3A-3F can be used for a specified purpose. The program may be downloaded, as the first information, from the storage medium 4 through the first information processor 1.

One of the second information processors 3A-3F may have a communication protocol which is different from that of the analyzer 2. In that case, the second information need be modified in e.g. data format in conformity with the communication protocol of the second information processor 3 before it is outputted from the analyzer 2 to the second information processor 3. In light of this, the first information may include a program to change the format of the second information to correspond to the communication protocol of the second information processor 3, and the first information may be downloaded into the analyzer 2 through the first information processor 1.

The PC 3A or the PDA 3B, owned by e.g. a patient who measures a particular component, can store the results of the concentration measurement obtained from the analyzer 2. Software for analyzing the measurement results and giving some appropriate advice or warning (comparable to instructions by a doctor) may be preinstalled in the PC 3A or the PDA 3B. Since it is preferable to finally receive instructions from a doctor based on the results of the concentration measurement, the owner of the analyzer 2 may need to transmit the results of the concentration measurement to a medical institution. When the PC 3A or the PDA 3B by itself is not able to transmit data to another terminal through telecommunication lines, the results of the concentration measurement are transmitted from the PC 3A or the PDA 3B to a server 6 via a communication apparatus 5 such as a mobile communicator. The transmission of the concentration measurement results may be performed by e-mail, for example. The server 6 may be a server by which the medical institution or the manufacturer/distributor of the analyzer 2 sets up their homepage, or e-mail server of the medical institution. Alternatively, the concentration measurement results obtained by the analyzer 2 may be managed by the mobile communicator 3D. In such a case, the measurement results may be transmitted from the mobile communicator 3D to the server 6. In these cases, the concentration measurement results are included in the second information which the PC 3A, the PDA 3B or the mobile communicator 3D receives from the analyzer 2.

With the game machine 3C, various kinds of games can be played by switching different kinds of game software or by downloading game programs by access to a homepage. The game machine 3C may be designed as a home-use machine or a portable machine. Game machines are mainly used by children. Recently, the number of children with diabetes is increasing. However, it is difficult to get children into the habit of measuring their blood glucose level at home. Therefore, to motivate children to measure the blood glucose level, a point or points may be given to children in accordance with the accomplishment of measurement on predetermined occasions for measuring the blood glucose level (degree of the acquirement of the habit) or in accordance with the degree of improvement on the blood glucose level. In accordance with the points which children gained, a special image which amuses children may be displayed on the display of the analyzer 2 or the game machine 3, or a strong character or a special weapon desired for a battle game may be made obtainable. Further, the ranking of the game scores may be made public on a homepage so that children are motivated to measure the blood glucose level to get points. The special weapon may be given as the second information from the analyzer 2. Alternatively, the data regarding the measurement results or the degree of accomplishment of measurement may be transmitted as the second information from the analyzer 2 to the server (homepage) 6 through the movable communicator 3D, and the special weapon may be downloaded from the server 6 to the game machine 3C through the communication apparatus 5 in accordance with the data.

The printer 3E is used to print out the second information stored in the analyzer 2. The printer 3E may belong to an individual or a medical institution that owns the analyzer 2. A patient who has neither the PC 3A nor the PDA 3B may make a hard copy of the measurement results of the blood glucose level using the printer 3E and attach the hard copy to a notebook, for example. Further, in visiting a medical institution, the hard copy may be handed to a doctor for receiving instructions. Also in a medical institution, a hard copy of data may need to be attached to a notebook for keeping the measurement data. In these cases, using the printer 3E, the measurement data, the time and date of measurement, the identification of the patient, the identification of the measurer (e.g. the ID number of the nurse) and the like are printed out as the second information.

The PC 3F is a personal computer used by a medical institution. When the blood glucose levels of several inpatients are measured using the analyzer 2, the measurement data may be inputted into the PC 3F for monitoring the blood glucose levels of the respective inpatients. In this case, similarly to the case of the PC 3A for personal use, the first information may be a program for modifying the data format of the second information in conformity with the communication protocol of the analyzer 3. The second information may be measurement data, for example.

In the information communication system X described above, it is possible to change the original function of the analyzer 2 or to add a new function to the analyzer 2 by downloading the first information into the analyzer 2 through the first information processor 1. Generally, the functions of the analyzer 2 can be halved, those directly related with concentration calculation, and those not directly related with concentration calculation.

The functional changes directly related with the concentration calculation may include the change of a calibration curve and change of a correction function of the measurement data. When the analyzer 2 is a simplified measurement apparatus for personal use, the measurement results may not correlate with the measurement results to be obtained by a medical institution utilizing a standard analyzing method. In this case, the first information may be downloaded to adjust the calibration curve or the correction function, the analyzer 2 can correct the measurement results to make them close to the measurement results by the standard analyzing method. As a result, the medical institution need not correct the measurement data transmitted thereto, whereby the burden on the medical institution is reduced. Additional items subject to modification may include the measurement range or certain measurement conditions (for example, a reaction time, in a case where the analyzer 2 operates according to a reaction-related method, or voltage to be applied, in a case where an electrochemical method is employed).

Examples of functions which are not directly related with the concentration calculation include (1) a function to input, and display or store the registration information of a user (patient) and the registration information of ameasurer (nurse) when the analyzer 2 is used for inpatients in a medical institution, (2) a function to change a user-specific critical measurement threshold (upper threshold value or lower threshold value) and further to give a warning when the measured value is not in the range defined by the threshold value, (3) a function to allow data input of the kind and amount of food eaten (caloric intake) and the amount of exercise (caloric expenditure) and further to give instructions in accordance with the input, and (4) in a case where the second information processor 3 is a game machine 3C, a function to display a particular image or to make a particular sound when the score is higher than a predetermined value.

As described above, the change or addition of functions are made possible through the downloading of the first information to the analyzer 2 via the first information processor 1. Specifically, the programs necessary for effecting the functions may be included in the first information in advance, and the relevant program is downloaded to the analyzer 2 through the first information processor 1.

As described above, the analyzer 2 may be a portable apparatus. In that case, the capacity of a storage (memory) is considerably limited as compared with that of a PC or a PDA. Therefore, it may not be possible to install all the programs included as the first information into the memory of the analyzer 2. Further, it is conceivable that a new function may be invented which the analyzer 2 can perform, or that a second information processor 3 of a new type may be developed. In this regard, it should be noted that the memory of the analyzer 2 includes a rewritable memory region. Thus, even when the capacity of the memory is limited, the owner of the analyzer 2 may select the functions to be performed with the analyzer 2 or select the kind of the second information processor 3 to be used. The function or the kind to be adopted can be changed in accordance with the first information downloaded from the storage medium 4 through the first information processor 1. In the case where the storage medium 4 is a database or an optical disk, such a new function of the analyzer or anew kind of second information processor 3 becomes usable by storing a program to realize the new function in the database or by preparing an optical disk storing a program to make the new kind of second information processor 3 usable. Therefore, the user need not obtain a new analyzer 2 provided with the new function or suitable for the new kind of second information processor 3. The user only needs to rewrite the existing program in the analyzer 2 or to add a new program.

Preferably, the communication protocol in conformity with the second information processor 3 selected by the user may be preinstalled in the analyzer 2. In this way, it is possible to eliminate the need to make a connection to the PC with a program to enable adjustment to the communication protocol of the second information processor 3, the connection being otherwise required to be made every time the second information is transmitted to a medical institution, for example.

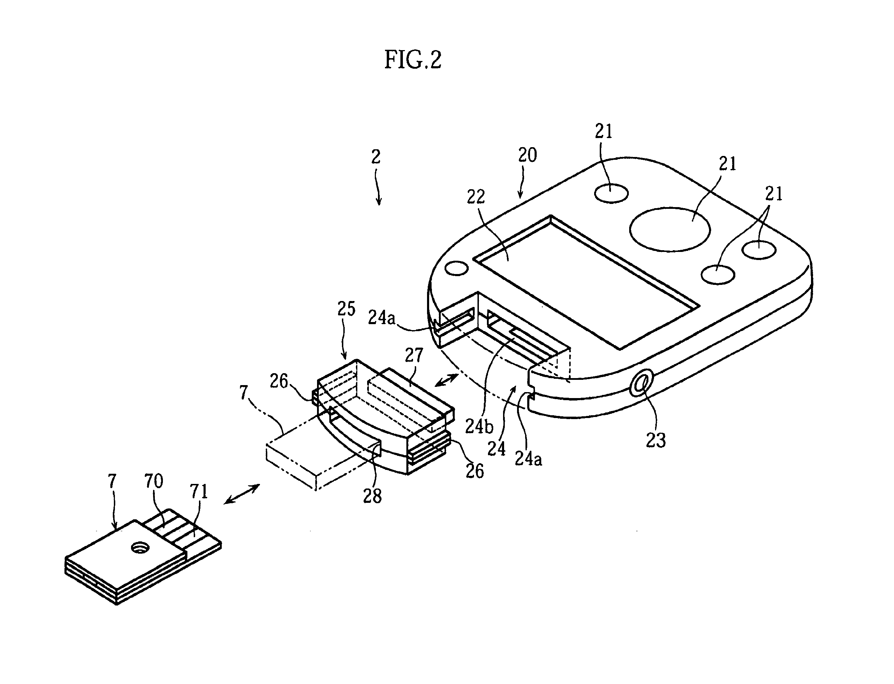

Next, with reference to Figs. 2-6, the function-addable/-modifiable analyzer 2 will be described below, together with an example of the first information inputted to the analyzer 2. As shown in Fig. 2, the analyzer 2 includes a main body 20 and a detection unit 25. The detection unit 25, removably attached to the main body 20, outputs data to the main body 20.

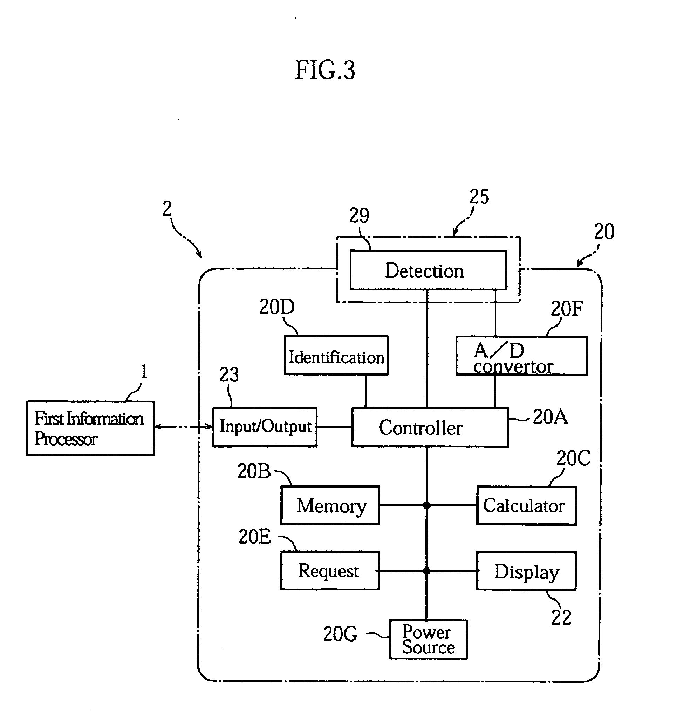

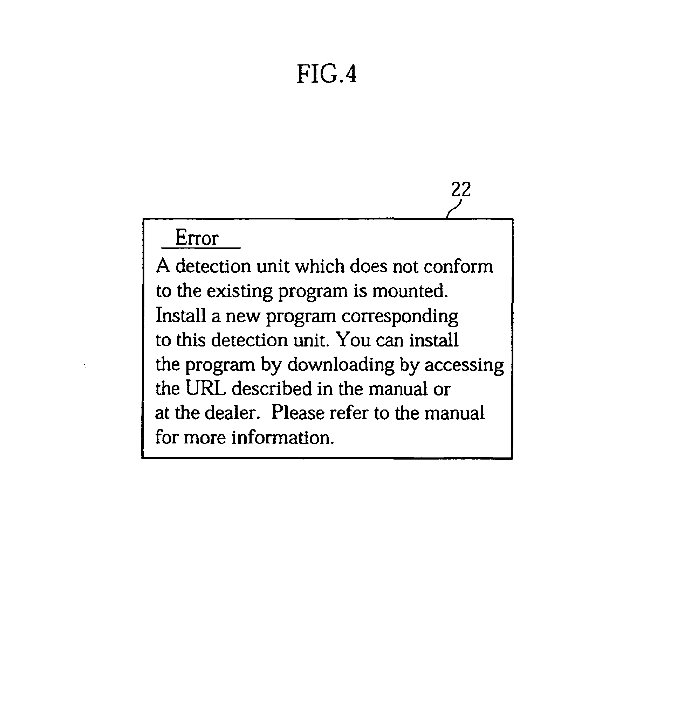

The main body 20 includes a plurality of operation buttons 21, a display 22, an input/output section 23 and a recess 24. The display 22, which displays analysis results or an error message, for example, may comprise an LCD. The input/output section 23 is provided formaking a connection to the first information processor 1 so that data is transmitted to or supplied from the first information processor 1 (See Figs. 3 and 4).

The recess 24 is provided for mounting the detection unit 25 therein. The recess 24 includes a pair of grooves 24a and a female connector 24b. The paired grooves 24a serve as a guide in inserting the detection unit 25 and also secure the detection unit 25. The female connector 24b is used for data transmission between the detection unit 25 and the main body 20 and power supply to the detection unit 25, while also functioning to secure the detection unit 25.

The detection unit 25 has a configuration generally corresponding to that of the recess 24. Specifically, the detection unit 25 includes a pair of projections 26 corresponding to the paired grooves 24a, and a male connector 27 for fitting in the female connector 24b.

As shown in Fig. 3, the main body 20 further includes a controller 20A, a memory 20B, a calculating section 20C, an identifying section 20D, a requesting section 20E, an A/D converter 20F and a power source 20G. The controller 20A, the memory 20B, the calculating section 20C, the identifying section 20D and the requesting section 20E are realized by a CPU, a ROM or a RAM, or a combination of these.

The controller 20A controls the operation of the memory 20B, the calculating section 20C, the identifying section 20D, the requesting section 20E and so on. The memory 20B stores programs for performing various operations such as calculation and also stores analysis results, for example. Based on an output from the detection unit 25, the calculating section 20C performs calculation required for the analysis of a particular component contained in a measurement object.

The identifying section 20D identifies the type (in accordance with the differences in measurement item or measurement method, for example) of the detection unit 25 mounted to the main body 20. The identifying section 20D may identify the type of the detection unit 25 when a button is operated by the user or may automatically identify the type of the detection unit 25 when the detection unit 25 is mounted to the main body 20. In the latter case, information corresponding to the type of the detection unit 25 is transmitted from the detection unit 25 to the identifying section 20D, whereby the identifying section 20D identifies the type of the detection unit 25.

The requesting section 20E determines whether or not proper calculation, which depends on the mounted detection unit 25, can be performed by utilizing the currently stored program, and the section requests for the installation of a new program when it is determined that the current program cannot deal with the calculation. The request for installation may be displayed on the display 22 as shown in Fig. 4, for example.

Analog data outputted from the detection unit 25 is converted to digital data by the A/D converter 20F. The A/D converter 20F may be incorporated in the detection unit 25. In that case, the detection unit 25 outputs digital data. The power source 20G may comprise a DC power source such as a dry cell or a rechargeable battery, to supply electric power necessary for operating the main body 20 and the detection unit 25. Alternatively, a separate power source may be built in the detection unit 25 so that the main body 20 and the detection unit 25 are driven by different power sources.

As shown in Figs. 2 and 3, the detection unit 25 further includes a mounting portion 28 and a detecting section 29. As clearly shown in Fig. 2, the mounting portion 28 is utilized for mounting an analyzing tool 7. The illustrated analyzing tool 7 is a biosensor including a pair of electrodes 70 and 71, a capillary (not shown) and a reagent layer (not shown). The reagent layer contains oxidoreductase, for example. By selecting an appropriate kind of oxidoreductase, it is possible to make the reagent layer to output a response corresponding to the concentration of an intended component such as glucose, cholesterol or lactic acid, for example. In the analyzing tool 7, sample liquid (measurement object) such as blood is introduced to the reagent layer by utilizing the capillary to form a reaction system in the liquid phase. When a voltage is applied (i.e., stimulation is given) to the reaction system by the paired electrodes 70 and 71, a response to the stimulation is outputted as electric current.

The detection section 29 obtains data from the measurement object for performing required calculation. In the case where the analyzing tool 7 having the above structure is used, the detection section 29 includes a pair of terminals (not shown) for coming into contact with the electrodes 70 and 71 of the analyzing tool 7. The paired terminals are electrically connected to the male connector 27 and hence connected to the electric circuit (the power source 20G and so on (See Fig. 3)) of the main body 20 via the male connector 27. Therefore, with the detection unit 25 having the paired terminals, it is possible to apply a voltage across the electrodes 70 and 71 of the analyzing tool 7 and also to output a response from the reaction system as electric current.

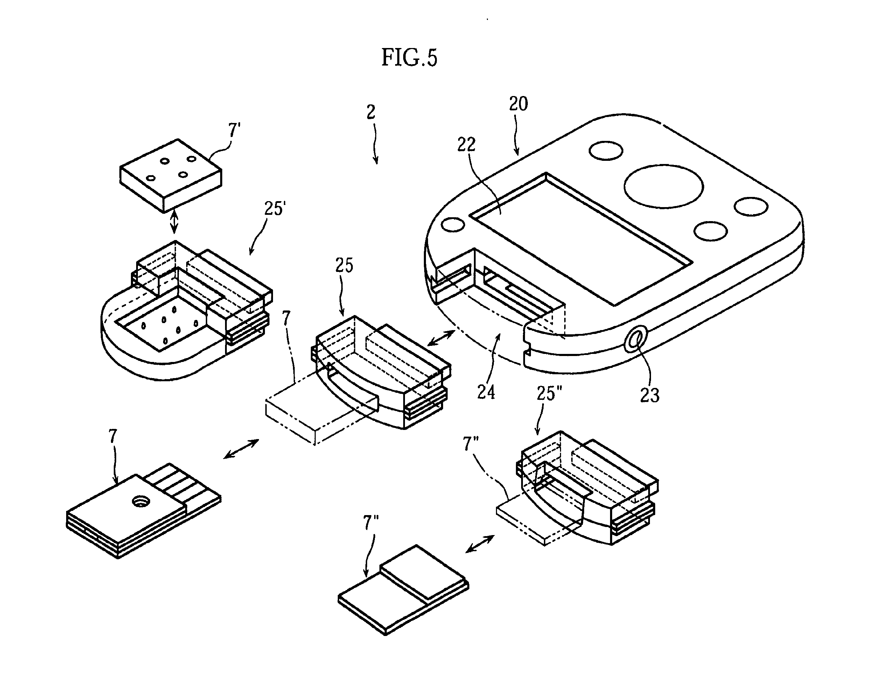

In the above analyzer 2, the detection unit 25 is removable from the main body 20, so that an old detection unit 25 can be replaced by a new one. For the same reason, a different detection unit or units 25 can be used in addition to the existing detection unit 25. As shown in Fig. 5, when a new detection unit or units are used additionally, the user may select a suitable one from the detection units 25, 25' and 25" of different kinds depending on the item to be measured. Fig. 5 shows a detection unit 25 utilizing a biosensor 7 for the analyzing tool, a detection unit 25' utilizing an ion-selective plate 7' for the analyzing tool, and a detection unit 25" utilizing a test piece 7" for the analyzing tool. It should be appreciated that the detection units 25, 25' and 25" and the analyzing tools 7, 7' and 7" are merely examples, and a detection unit for performing analysis in a noninvasive manner may be used.

For additional use or modification of detection units, a new calculation program appropriate for the new detection unit may need to be installed into the memory 20B of the analyzer 2. The necessity of installation is determined by the identifying section 20D. Specifically, the identifying section 20D identifies the type of the mounted detection unit 25 (25', 25") and determines whether or not the analysis for the detection unit 25 (25', 25") can be performed by the program stored in the memory 20B. When the identifying section 20D determines that the analysis cannot be performed, the requesting section 20E displays a request for installation of a program at the display 22, as shown in Fig. 4.

When such a request is displayed, a new program is installed via the input/output section 23 in accordance with the procedure described with reference to Fig. 1, so that a new function is added to the analyzer 2 or the existing function is modified.

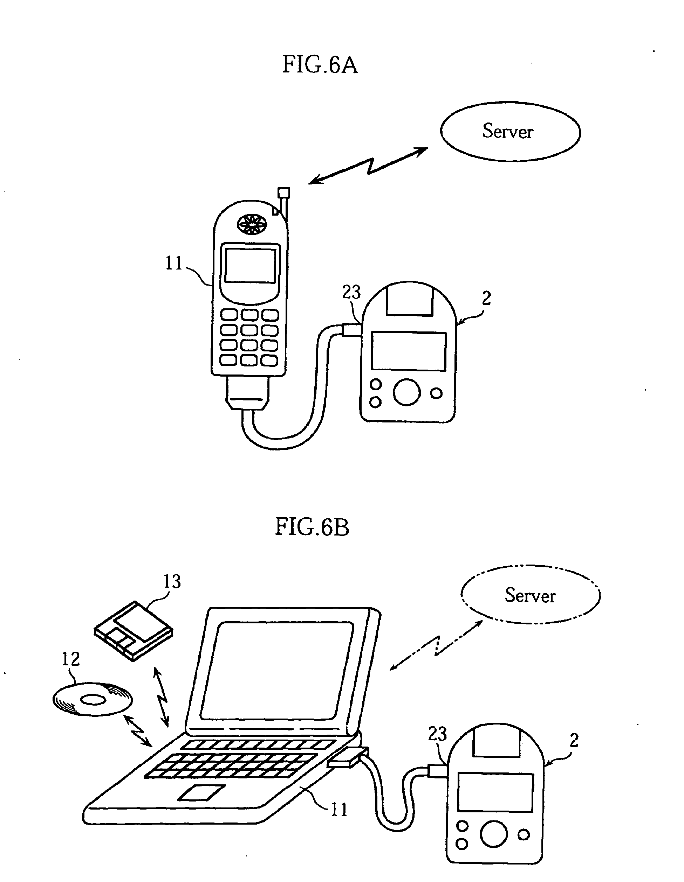

As shown in Fig. 6A, the installation of a new program onto the analyzer 2 may be performed by connecting a mobile communicator 10 as a first information processor 1 to the analyzer 2 utilizing the input/output section 23 of the main body 20, for example. In this case, the new program may be downloaded from a database of a server or a homepage utilizing the Internet. Alternatively, as shown in Fig. 6B, a personal computer 11 as a first information processor 1 may be connected to the analyzer 2 utilizing the input/output section 23 of the main body 20 so that a new program can be installed utilizing the personal computer 11. In this case, the program to be installed may be stored, in advance, in a built-in hard disk of the personal computer or in an external memory (e.g. a compact disc 12 or a flexible disk 13).

In installing a new program, the existing program stored in the memory 20E may be uninstalled or may be left as it is. Whether or not the existing program is to be uninstalled may be determined by the user or may be determined by the apparatus depending on the remaining capacity of the memory 20E.

## Claims

1. An information communication system comprising a storage medium in which target information is stored in advance, an information processor for inputting the target information from the storage medium, and an analyzer for inputting the target information from the information processor;
wherein the target information from the storage medium is inputted into the analyzer through the information processor.

2. The information communication system according to claim 1, wherein the information processor is a personal computer, a set-top box or a mobile communicator.

3. The information communication system according to claim 1, wherein the storage medium is a database accessible through telecommunication lines, an optical disk or a magnetic disk.

4. The information communication system according to claim 1, further comprising an additional information processor into which information from the analyzer is inputted and which performs a predetermined operation in accordance with the information.

5. The information communication system according to claim 4, wherein the target information is information necessary for realizing the operation of the additional information processor.

6. The information communication system according to claim 4, wherein the target information includes a program for causing said information from the analyzer to correspond to a communication protocol of the additional information processor before said information is outputted.

7. The information communication system according to claim 4, wherein the additional information processor is selected from additional information processors of different kinds;
wherein the additional information processors of different kinds include at least one selected from a mobile communicator, a PDA, a personal computer, a portable game machine, a home-use game machine and a printer.

8. The information communication system according to claim 4, wherein the information processor and the additional information processor are realized by a same device.

9. The information communication system according to claim 1, wherein the target information includes a calculation program necessary for analysis of an object at the analyzer.

10. The information communication system according to claim 1, wherein the target information includes a program for realizing a function which is necessary for an operation of the analyzer but is not directly related with calculation required for analysis of an object.

11. The information communication system according to claim 1, wherein the analyzer includes a rewritable memory, the target information being stored in the memory by overwriting content of the memory.

12. The information communication system according to claim 1, wherein the analyzer measures concentration of a particular component in a measurement object.

13. The information communication system according to claim 12, wherein the analyzer measures glucose concentration in blood.

14. The information communication system according to claim 1, wherein the analyzer includes a main body, and a detection unit removably attached to the main body for outputting information to the main body;
wherein the main body includes a calculating section for performing calculation necessary for analyzing a particular component contained in a measurement object;
wherein the detection unit includes a detection section for obtaining calculation information necessary for the calculation from the measurement object.

15. The information communication system according to claim 14, wherein the detection section functions to provide stimulation for the measurement object and obtain the calculation information as a response to the stimulation.

16. The information communication system according to claim 14, wherein the main body further includes a memory for storing a program necessary for the calculation and an inputting section for inputting a new calculation program from outside;
wherein the new calculation program is stored in the memory.

17. The information communication system according to claim 16, wherein the main body further includes a detection-unit-identifying section for identifying a type of the detection unit mounted, and an install requesting section for requesting installation of a new calculation program based on the identification by the detection-unit-identifying section.

18. The information communication system according to claim 16, wherein the detection unit has a structure allowing an analyzing tool for retaining the measurement object to be mounted.

19. The information communication system according to claim 1, wherein the analyzer is a portable device.
